Europäisches Patentamt

(19) European Patent Office

Office européen des brevets

(11) Publication number : **0 466 383 A1**

# (12) EUROPEAN PATENT APPLICATION

(21) Application number : **91306000.0**

(51) Int. Cl.⁵ : **A61L 25/00**

(22) Date of filing : **02.07.91**

(30) Priority : **09.07.90 US 549797**

(43) Date of publication of application :
**15.01.92 Bulletin 92/03**

(84) Designated Contracting States :
**DE DK ES FR GB IT SE**

(71) Applicant : **BAUSCH & LOMB INCORPORATED**
**One Lincoln First Square PO Box 54**
**Rochester New York 14601-0054 (US)**

(72) Inventor : **Bowyer, Barry L.**
**1754 Grove Drive**
**Clearwater, Florida 34619 (US)**
Inventor : **Robin, Jeffrey**
**100 Redwood Lane**
**Barrington, Illinois 60010 (US)**

(74) Representative : **Allam, Peter Clerk et al**
**LLOYD WISE, TREGEAR & CO. Norman House**
**105-109 Strand**
**London WC2R 0AE (GB)**

(54) **A collagen medical adhesive and its uses.**

(57)    An adhesive composition suited for surgical applications which consists essentially of an aqueous solution of natural collagen which has a melt index temperature within the range of 35°C to 45°C.

EP 0 466 383 A1

Adhesives which can be used in surgical applications have been taught since the late 1940's. The first report of a surgical adhesive was published in 1949 concerning Tassman's use of fibrin coagulum to fix keratoplasty wounds, to hold cataract surgery incisions and to repair corneal lacerations. This adhesive did not enjoy success due to its susceptibility of enzyme degradation in in vitro surgical applications. Fibrin as a surgical adhesive was almost completely abandoned in the early 1950's.

In the early 1960's the National Health Institute and Battelle Memorial developed a adhesive based upon resorcinol crosslinked gelatin (specifically a mixture of gelatin, resorcinol and formaldehyde, sometimes referred to as "GRF"). While gelatin can be considered biocompatible with many tissues, the other additives of this mixture pose serious cytotoxity concerns. Furthermore, the adhesives employing this chemistry proved complex to prepare and subject to inconsistent adhesive characteristics. While the adhesive capacity required for applications in the heart could be achieved, it was not achieved in all instances.

Concurrent with the GRF research cyanoacrylates became available and their application in surgical applications was investigated. The adhesive characteristics of these materials are excellent. However, the materials are generally quite rigid, they are impermeable to nutrients essential to tissue healing and their byproducts, formaldehyde and cyanide, are toxic to tissue cells. Furthermore the materials do not bioerode so they may have to be removed at some later date. While the materials are used in a small number of surgical applications, such as closing small perforations, the United States Federal Food and Drug Administration has not approved of their use. Furthermore, due to their basic plastic like characteristics they cannot be used to close incisions.

In the 1970's investigators in Europe developed an adhesive composed of human fibrinogen and bovine thrombin which is commercially available in Europe as Tisseel[r] (from Immuno Ag, Vienna, Austria). Since the commercial product is made from pooled human serum the possibility of viral infection is present and limits the desirability of the adhesive. This adhesive is a multi-component system which is difficult to prepare in order to provide consistent results and one of its components, fibrinogen, degrades rapidly when used to bond living tissues together.

Recently, a polyphenolic proteinaceous adhesive derived from molluscs has been developed. This adhesive is a two component system which is difficult to prepare consistently and produces widely varying adhesive characteristics.

Other surgical or medical adhesives have been reported in the patent literature. For instance, U.S. Patent No. 4,035,334 describes a medical adhesive comprised of two epoxy ethyl-alpha-cyanoacrylate, poly-N-vinyl ether and 1-4, bis (p-toluidino)-anthraquinone. While this medical adhesive is an improvement over prior cyanoacrylates, it does still require the use of diisocyanate chemistry to polymerize and thus still presents potentially toxic chemistry to the tissue being joined. Furthermore, the polymerized material is not erodible and must be removed at some point in time. Furthermore, the materials which comprise this adhesive chemically bond to tissue which can damage the tissue.

Collagen materials have been widely considered useful in medical applications. For instance, U. S. Patent No. 4,760,131 describes a wound healing composition comprising fibrillar collagen, heparin and granulated platelets or platelet releaseate. However, this composition is meant to act as a wound dressing; it has no adhesive properties and cannot be considered a wound or incision closing agent. Reported medical adhesives include a crosslinked gelatin which had been researched extensively in the mid to late 1960's. See, for instance, the October 1966 issue of Surgery, pages 857-861 entitled "The use of Cross-Linked Gelatins - a Tissue Adhesive to Control Hemorrhage from Liver and Kidney". It should be noted that this material was not intended close wounds. Furthermore, the procedures reported uses a gelatin-resorcinol mixture crosslinked with formaldehyde as the adhesive. The use of a medical material that includes or produces formaldehyde is unfeasible.

It is an object of the invention to provide a medical adhesive which allows for the closure of wounds or surgical incisions and allows for the augmentation or replacement of sutures. Furthermore, it is an object of this invention to provide an adhesive which will promote healing of the wound and will not cause localized stresses on the incision or wound. Further objects are to provide an adhesive which does not degrade in its adhesives rapidly, which does not polymerize so rapidly that the tissues being joined cannot be manipulated into their desired configuration and which does not require complex preparation just prior to its use. Furthermore, it is an object of the present invention to provide an adhesive which is permeable to nutrients essential to healing of the wound or incision.

## BRIEF DESCRIPTION OF THE FIGURES

Figure 1 is a scanning electron microscope photograph of the cross-section of a rabbit cornea tissue sample bonded together with adhesive of the present invention.

Figure 2 is a scanning electron microscope photograph of the cross-section of a rabbit cornea tissue sample which had been bonded with a 12.5 weight percent collagen adhesive of the present invention after being pulled apart.

Figure 3 is the scanning electron microscope photograph of a cross section of a rabbit cornea tissue sample glued together with a 20 weight percent collagen solution.

## SUMMARY OF THE INVENTION

The present invention relates to a medical adhesive which can be used as a material useful in closing wounds and/or surgical incisions. The composition consists essentially of an aqueous solution of collagen that has a melt index in the range of 35°C to 45°C.

In general, this characteristic can be achieved by controlling the crosslink density of the collagen. The viscosity can be controlled to some degree by varying the concentration of the total amount of collagen in the aqueous solution. Preferably, this concentration is within the 5 to 30 weight percent range.

The present invention allows one to provide an adhesive composition with a set of characteristics which may uniquely be suited to a specific medical procedure since by blending crosslinked and noncrosslinked collagen one can provide a material with a given viscosity, adhesiveness, melt index temperature, setting temperature and transparency.

The invention in a preferred embodiment is a medical adhesive useful in closing wounds or surgical incisions which consists essentially of an aqueous solution of naturally occurring collagen wherein said solution has the following characteristics: 1) a melt index within the range of 35°C to 45°C, 2) a viscosity which allows the material when heated above its melt index temperature to flow readily, and 3) an ability to adhere to tissues. These characteristics are obtained by cross-linking natural collagen to a specified degree at a given concentration. Control of the degree of crosslinking can best be achieved by mixing a solution of crosslinked collagen with a solution to a relatively noncrosslinked natural collagen (collagen which has been extracted and purified but not undergone any processes which increased the cross-link density of the material).

Increasing the crosslink density of the collagen material can be achieved by a number of means. For instance, the collagen can have its crosslink density increased by exposure to ultraviolet irradiation. Collagen can also be crosslinked by thermal means. For instance, collagen solution can be dried and then heated above 100°C to a defined temperature for a controlled length of time which will result in a specific crosslinked density. After this step, the crosslinked collagen is then put into solution where it exhibits the same characteristics as the noncrosslinked natural collagen solution with the exception that its melt index is higher than a noncrosslinked collagen and its viscosity above its melt index temperature is slightly higher.

Melt index is defined for the purpose of this application to be the temperature mid-point at which a collagen solution changes from being relatively nonflowable to flowable. In practical terms, one sees the collagen solution go from a very thick nonflowable material to a liquid which flows quite readily (typically the viscosity of the solution goes from being immeasurable to less than 5000 centipoise when the solution is heated above its melt index temperature). This transition occurs over a relatively narrow temperature band (typically less than about 8 to 10 degrees Centigrade). It is thought that this phenomenon occurs because as the solution temperature increases the internal hydrogen bonding of the triple helix of the collagen decreases and the molecule unwinds. Thus, the collagen molecule loses much of its secondary structural characteristics and becomes more flowable. By increasing the covalent crosslinked density across the strands one can increase the triple helices integrity and raise its melt index.

The collagen material used in the present invention can be purified from any number of sources. However, it is preferred that the collagen material be Type I collagen. The preferred source of the collagen is porcine sclera.

A typical means of obtaining such materials is by treatment of the sclera of an animal eye. The eye is thoroughly cleaned of its internal coatings, the residual tissue is removed and the sclera is cleaned and isolated. The isolated sclera is then cut into small pieces, then rinsed with distilled water and transferred to a solution of alkaline hydroxide in a saturated solution which digests the sclera typically for an extended period (typically 48 hours). The solution is then decanted, the tissues are ph neutralized to within a range of 6.0 to 7.0 (typically with boric acid) and stirred. The solution is then diluted with water and dialyzed to remove all of the acid counter ion. The solution is then put into a 0.5 to 1.0 molar solution of an organic acid and allowed to stand, under refrigeration, for up to 3 days. The solution is homogenized, centrifuged and allowed to stand for 1 further day at low temperature. The sample is then dialyzed against a buffer system bringing the solution to a pH of 4.5 to 7.5. The material is then further centrifuged to remove any residual particulate matter.

The properties of the adhesive, i.e. its adhesiveness, viscosity, melt index temperature and transparency can be controlled by means of controlling the crosslink density of the collagen, the collagen concentration and the source of the collagen. Thus one can according to the invention provide an adhesive with a specific set of characteristics for a given medical procedure.

One method for controlling the crosslink density of the collagen is to blend solutions of densely crosslinked collagen with solution of noncrosslinked collagen. Typically, the total collagen content of the medical adhesive will be in the range of 5 to 35 weight percent of the solution total. The ratio of densely crosslinked collagen to relatively noncrosslinked collagen can vary from 10 to 1 to 1 to 1. Of course, it should be recognized as well that this ratio may rely upon the degree of crosslinked density in the densely crosslinked collagen material.

The starting purified collagen solution material may be used as the relatively noncrosslinked collagen solution, although it may be crosslinked to a degree. The essential concept is to have two solutions with different crosslink densities in order to blend the two to produce the precise solution characteristics with respect to viscosity and melt index.

It is also possible to obtain a medical adhesive of the present invention by controlling the degree of crosslinking of only one collagen solution and using this solution as a medical adhesive. Presently, the most convenient approach is to blend collagen solutions.

The present invention is useful as a medical adhesive in a wide range of surgical procedures including many ophthalmic procedures such as cataract surgeries, epikeratophakia, corneal perforations, corneal transplants and other ophthalmic surgical procedures.

Specifically, it is believed the adhesive will be useful in cataract extraction. Recent trends in cataract surgery especially the trend to smaller and posterior incisions being used more posterior than previous practices appear to make the use of adhesive more advantageous. Such techniques provide incisions which are flap-like in form and require less compressive force to close. The adhesive could also be used intraocularly in complicated extracapsular cataract extractions where there has been a rent or rupture in the posterior capsule.

The adhesive will also be useful in corneal transplants. The standard method presently used employs up to sixteen individual sutures. The adhesive may reduce the degree of postoperative astigmatism and allow for more rapid visual rehabilitation by reducing the number of sutures.

Lamellar Keratoplasty, which is a partial thickness corneal transplant performed for the treatment of specific eye diseases where a full thickness transplant is not indicated, can benefit from the use of the adhesive. Layering of the adhesive along the lamellar resection may allow for better wound closure than can be presently accomplished. The benefits of the adhesive outlined above will also be available in other surgical procedures such as retinopexy and ophthalmic plastic surgery.

The adhesive is used by first heating the aqueous solution of collagen above its melt index temperature so that it will readily flow. The material is then placed on the surface of the tissue planes to be adhered, and then the tissue is appropriately positioned. As the adhesive cools to body temperature it thickens into an effective adhesive.

In addition to acting as an adhesive, the material may also be employed to deliver pharmacological agents. Pharmacological agents may be incorporated into the formulation in order to help control the rate of healing at the wound site. For instance, growth factors could be added to the adhesive to accelerate healing. Alternately, factors such as cortistearoids or antimetabolites could be added to slow wound healing. Antibiotics may be added to the adhesive as well. The use the adhesive in conjunction with pharmacological agents allows one to deliver to the precise wound site the pharmacologic agent. One can thus use a smaller amount of pharmacologic.

The following examples illustrate the invention in certain aspects but do not fully delineate the scope of the invention.

Example 1

A 35 weight percent solution of naturally occurring collagen was prepared from a porcine sclera source. A portion of the solution was dried and heated to 145°C for 60 minutes to produce densely crosslinked collagen material. The material was reconstituted to a specified concentration. A second portion was similarly treated for 15 minutes at 145 degrees Celsius, and served as a relatively noncrosslinked sample.

A mixture comprising 5 weight percent of the relatively non-crosslinked material and 95 weight percent of the densely crosslinked material was made and diluted to various total collagen concentrations (12.5 weight percent, 15 weight percent, 20 weight percent and 30 weight percent collagen).

Freshly harvested rabbit corneal tissue was cut into standard size samples (about 5mm x 10mm) which were then bonded using the adhesive compositions which had been heated to more than about 38°C to 45°C and then allowed to cool. The bonding strength of the samples, in grams, was measured at specified periods after the tissue samples were initially joined with the adhesive. The following results were achieved as shown in Table I. The results are reported in grams per standard area of sample.

## TABLE I

### Bonding Strength (gm/std sample)

| wt% collagen | 5 minutes | 15 minutes | 30 minutes |
|---|---|---|---|
| 12.5 | 1 | 1 | 4.0 |
| 15 | 11 | 14 | 27 |
| 20 | 10 | 11 | 11 |
| 25 | 9 | 23 | 10 |
| 30 | 8 | 6 | 10 |

Example 2

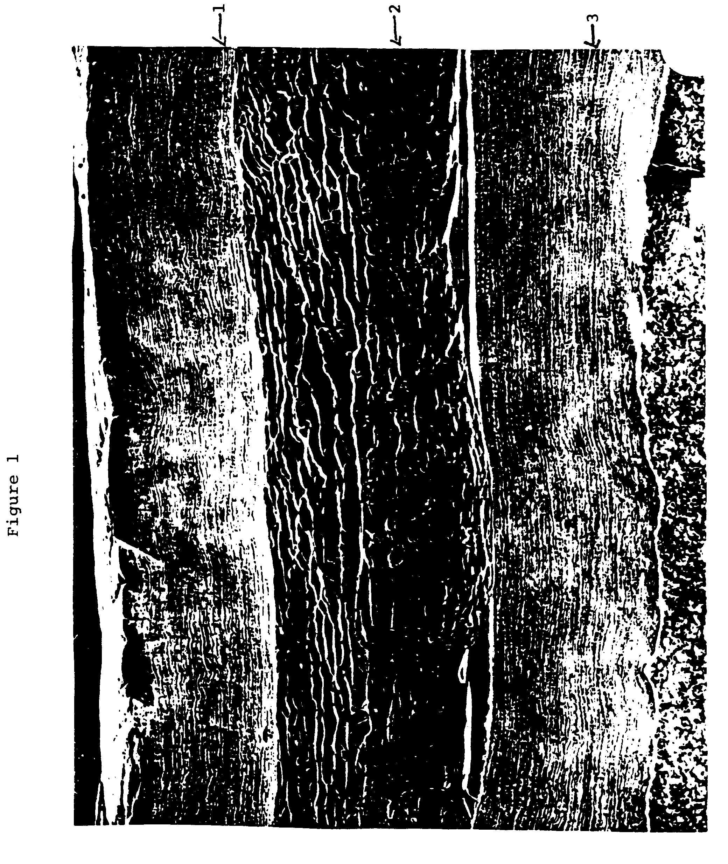

The 12.5 wt% total collagen medical adhesive as described in Example 1 was used to adhere rabbit corneal tissue samples together. Scanning electron microscopy was used to take pictures of the samples in various stages of testing the adhesive capacity of the glue. Figure 1 shows the tissue samples 1, 3 glued together with the adhesive of the present invention 2.

The samples glued together with 15 wt% collagen adhesive showed little disruption of the tissues being bonded, indicating that the strength of the tissues exceeded the strength of the adhesive bond. Samples adhered with the 20 wt.% collagen adhesive displayed significant damage to the tissue upon adhesive failure of the sample.

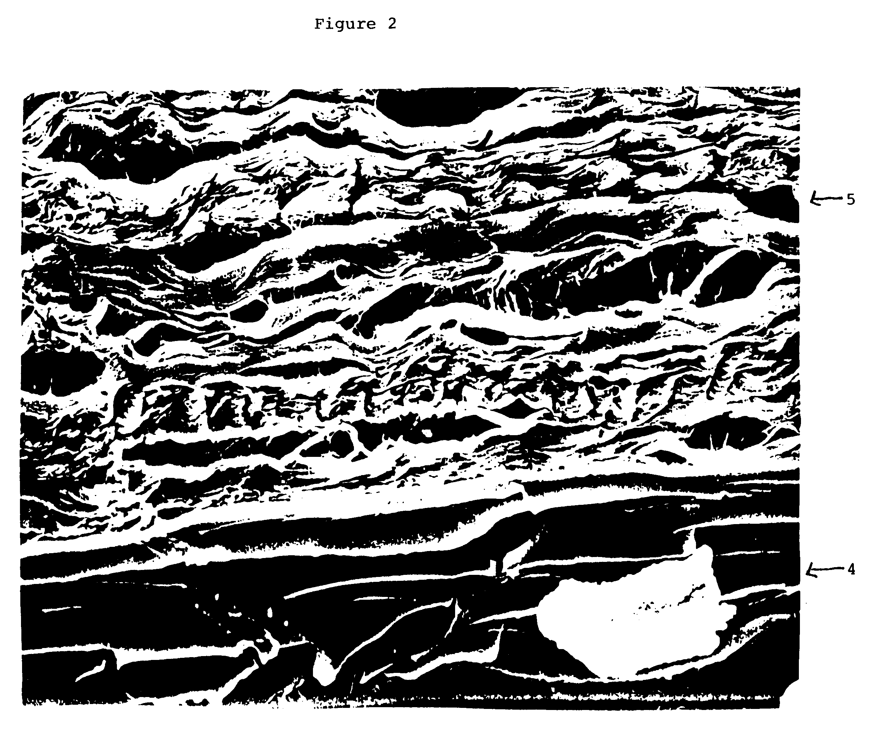

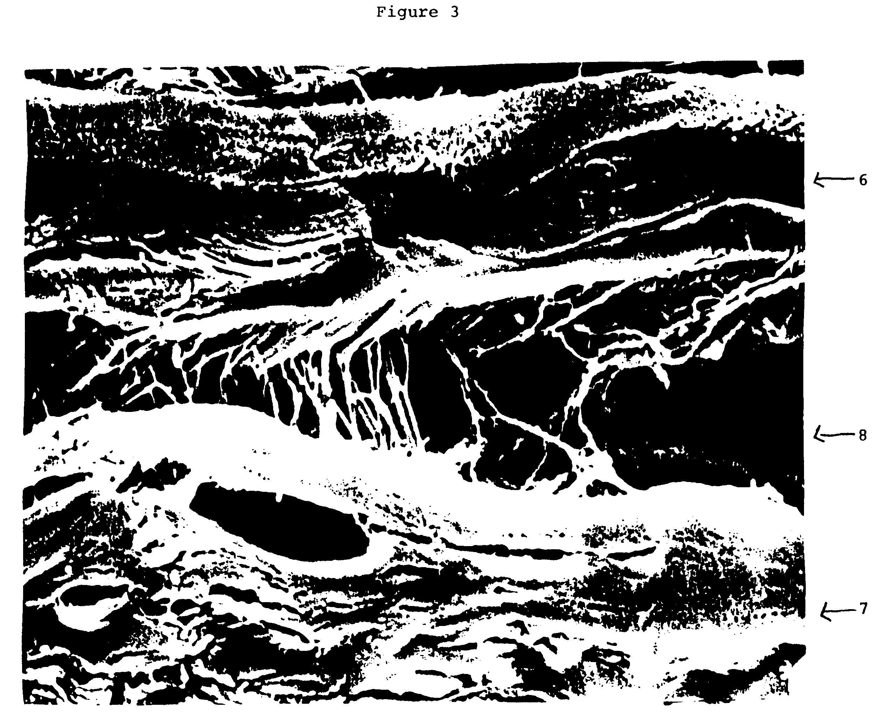

Figures 2 and 3 show the SEM of the failed samples using 12.5 wt.% and 20 wt.% collagen, respectively. Figure 2 shows that the point of adhesive failure was in the adhesive layer 4 and that the tissue 5 showed very little disruption. The degree of magnification in Figures 2 and 3 is greater than that used to generate Figure 1.

Figure 3 shows that when a 20 weight percent collagen adhesive was used the point of adhesive failure 8 was within the tissue sample 4, 6. These results indicate that with the 20 wt.% collagen adhesive the strength of the adhesive bond was greater than the strength of the tissue.

From these results, it is clear that the strength of the adhesive can be designed for a particular medical application.

COMPARATIVE EXAMPLE

Tissue samples were bonded together according to the method used in Example 1 using a collagen adhesive made according to the present invention which had a collagen content of 20 weight percent and a melt index of 35 degrees Centigrade. The collagen adhesive was heated to about 50 degrees Centigrade, applied to the surfaces of the tissue to be bonded and the tissue samples were then placed together. Samples were also bonded together using Tisseel[r] according to the directions indicated by the manufacturer. The samples were then tested for their adhesive bonding strength by applying weight to the samples in a fashion to create shear across the bonded area. The bond strength was taken as the weight required to cause failure in the sample. The bonding strength of the samples in grams was measured at specified times from preparation. The results are reported in Table 2.

## TABLE 2

### Comparative Bonding Strength (gm/std.sample)

| Time(minutes) | Tisseel[r] | Collagen Adhesive |
|---|---|---|
| 5 | 8 | 25 |
| 15 | 20 | 35 |
| 30 | 20 | 60 |

As can be appreciated from the results, the adhesive strength of the collagen adhesive is significantly

EP 0 466 383 A1

higher than the commercially available Tisseel[r] medical adhesive.

## Claims

1. An adhesive composition suited for surgical applications, comprising an aqueous solution of natural collagen which has a melt index temperature within the range of 35°C to 45°C.

2. An adhesive composition according to Claim 1 which has a viscosity within the range of 0.1 to 5000 centipoise above its melt index temperature.

3. An adhesive composition according to Claim 1 or Claim 2, wherein the melt index temperature of the aqueous solution of collagen is in the range of 40°C to 45°C.

4. An adhesive according to any preceding claim, comprising a blend of densely crosslinked collagen and noncrosslinked collagen.

5. An adhesive according to Claim 4, wherein the densely crosslinked collagen is achieved by thermal crosslinking means.

6. An adhesive according to Claim 4 or Claim 5, wherein the noncrosslinked collagen comprises either 5, 15, 20, 25 or 50 weight percent of the total collagen content of the composition.

7. An adhesive composition according to any preceding claim, wherein the total collagen content of the solution is in the range of 5-30 weight percent, preferably in the range of 20 and 30 weight percent.

8. A method for closing wounds comprising the step of contacting at least one surface of a wound with an adhesive composition according to any preceding claim.

9. A method for promoting healing of wounds which consists of contacting at least one surface of a wound with a composition which consists essentially of a solution of naturally occurring collagen in water with cultured epithelium cells dispersed throughout said solution, wherein said composition has a melt index between 35 and 45 degrees Celsius, and a viscosity, when heated above said melt index temperature of between 0.1 and 5000 centipoise.

10. A method for promoting healing of wounds comprising the step of contacting at least one surface of a wound with a composition which is an aqueous solution consisting essentially of purified, naturally occurring collagen, water, and growth factors, wherein said solution has a melt index between 35 and 45 degrees Celsius, and a viscosity when heated above said melt index temperature of between 0.1 and 5000 centipoise.

6

Figure 1

Figure 2

Figure 3

EP 0 466 383 A1

**European Patent Office**

# PARTIAL EUROPEAN SEARCH REPORT

which under Rule 45 of the European Patent Convention shall be considered, for the purposes of subsequent proceedings, as the European search report

Application Number

EP 91 30 6000

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 330 344 (CEDARS-SINAI MEDICAL CENTER)<br>* Column 7, lines 28-30 * | 1 | A 61 L 25/00 |
| | | | |
| | | | **TECHNICAL FIELDS SEARCHED (Int. Cl.5)** |
| | | | A 61 L |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely : 1 - 7

Claims searched incompletely :

Claims not searched : 8-10

Reason for the limitation of the search:

Method for treatment of the human or animal body by surgery or therapy (see article 52(4) of the European Patent Convention).

| Place of search | Date of completion of the search | Examiner |
|---|---|---|
| THE HAGUE | 09-10-1991 | PELTRE CHR. |